# EUROPEAN PATENT SPECIFICATION

(11) **EP 2 893 923 B1**
(45) Date of publication and mention of the grant of the patent: **24.11.2021**
(21) Application number: 13834905.5
(22) Date of filing: 06.08.2013
(51) Int. Cl.: A61P 43/00, A61K 9/51, A61K 47/26, A61K 8/25, A61Q 19/08, A61K 8/02, A61K 9/20, A61K 31/702, A61K 8/49, A61K 8/73, A61K 8/60

(54) **RELEASE OF SUBSTANCES INTO SENESCENT CELLS**
FREIGABE VON STOFFEN IN SENESZENTE ZELLEN
LIBÉRATION DE SUBSTANCES DANS DES CELLULES SÉNESCENTES

(30) Priority: 04.09.2012 ES 201231370
(43) Date of publication of application: 15.07.2015
(73) Proprietor: Universidad Politécnica De Valencia (UPV), 46022 Valencia (ES); Consejo Superior De Investigaciones Científicas (CSIC), 28006 Madrid (ES); Centro de Investigación Biomédica en Red (CIBER), 28029 Madrid (ES); Universidad Autónoma De Madrid (UAM), 28049 Madrid (ES)
(72) Inventor: MARTÍNEZ MÁÑEZ, Ramón, 46022 Valencia (ES); MURGUIA IBÁÑEZ, José Ramón, 46022 Valencia (ES); PERONA ABELLÓN, Rosario, 28006 Madrid (ES); AGOSTINI, Alessandro, 46022 Valencia (ES); MONDRAGÓN MARTÍNEZ, Laura, 50018 Zaragoza (ES); MORENO TORRES, Marta, 46022 Valencia (ES); MANGUAN GARCÍA, Cristina, 46010 Valencia (ES); MARCOS MARTÍNEZ, María Dolores, 46022 Valencia (ES); SOTO CAMINO, Juan, 46022 Valencia (ES); SANCENÓN GALARZA, Félix, 46022 Valencia (ES)
(74) Representative: Pons
(86) International application number: PCT/ES2013/070581
(87) International publication number: WO 2014/037596

(56) References cited:
- WO-A1-2011/093907
- ANDREA BERNARDOS ET AL: "Enzyme-Responsive Intracellular Controlled Release Using Nanometric Silica Mesoporous Supports Capped with "Saccharides"", ACS NANO, vol. 4, no. 11, 23 November 2010 (2010-11-23), pages 6353-6368, XP055233896, US ISSN: 1936-0851, DOI: 10.1021/nn101499d
- AGOSTINI, A. ET AL.: 'Targeted cargo delivery in senescent cells using capped mesoporous silica nanoparticles' ANGEWANDTE CHEMIE (INTERNATIONAL ED. IN ENGLISH) vol. 51, no. 42, 15 October 2012, GERMANY, pages 10556 - 10560, XP055233895
- GARY-BOBO, M. ET AL.: 'Cancer therapy improvement with mesoporous silica nanoparticles combining targeting, drug delivery and PDT.' INTERNATIONAL JOURNAL OF PHARMACEUTICS vol. 423, no. 2, 28 February 2012, NETHERLANDS, pages 509 - 515, XP055232901
- GARY-BOBO, M. ET AL.: 'Multifunctionalized mesoporous silica nanoparticles for thetreatment of retinoblastoma: Drug delivery, one and two-photon photodynamic therapy.' INTERNATIONAL JOURNAL OF PHARMACEUTICS vol. 432, no. 1, 21 April 2012, pages 99 - 104, XP028510752
- BERNARDOS, A. ET AL.: 'Enzyme-responsive intracellular controlled release using nanometric silica mesoporous supports capped with ''saccharides''.' ACS NANO vol. 4, no. 11, 23 November 2010, UNITED STATES, pages 6353 - 6368, XP055233896
- None

## Description

The present invention relates to nanodevices for the controlled release of pharmaceuticals, biopharmaceuticals and cosmetics. Therefore, the invention lies within the fields of the pharmaceutical and the cosmetic industries.

### STATE OF THE ART

Normal somatic cells invariably enter a state of irreversibly arrested growth and of altered functions after a finite number of divisions, called cell senescence. Senescent cells show a radically altered phenotype which is believed to harm the function of tissues and to predispose them to the development and progression of diseases. Although the immune system destroys many senescent cells, it becomes much less effective at this task with ageing. Consequently, senescent or "death-resistant" cells accumulate in tissues, thus accelerating ageing and the development of diseases. Furthermore, the accumulation of senescent cells alters the behaviour of neighbouring cells, favours degradation of the extracellular matrix, reduces the source of cells capable of suffering mitosis and stimulates cancer.

There is a large number of pathologies associated with accelerated cell ageing such as Werner's syndrome of adult progeria (WS), Hutchinson-Gilford (HGS) and Rothmund Thompson syndrome. In WS or RTS erosion of the telomeres in most tissues can be observed, even if telomerase is not the main cause of the disease. Other diseases are more related to accelerated ageing in specific tissues, such as Dyskeratosis Congenita (DS) or Idiopathic Pulmonary Fibrosis (IPF). In these conditions, cells' capacity to replicate is damaged due to a defective activity of the telomerase in the stem cell compartment of tissues with high cell regeneration such as the skin, pulmonary epithelium and bone marrow. A frequent secondary effect in these diseases is the induction of cancer, especially of those cancers which imply a shortening of the telomeres. To combat this problem it is fundamental to have strategies to prevent, replace or eliminate senescent cells. In particular, the design of these therapies would contribute to the treatment of accelerated cell ageing and could stimulate the idea that rejuvenation is possible.

A first approach to achieve the objective of eliminating senescent cells would be related to the development of selective distribution systems capable of releasing their load in these particular cells.

Traditional release systems are based on polymers which normally do not respond to stimuli but rather release their load through controlled processes by diffusion and degradation of a polymeric container. On a separate note, nanotechnology has proven to be an innovative approach in therapies. Pharmaceutical release systems capable of releasing active molecules in certain cells in a controlled manner have received much attention of late, such as polymeric microcapsules, dendrimers, micelles and nanoparticles. Alternatively, mesoporous silica nanoparticles (MSN) have been used extensively in recent years as reserves for the storage of drugs due to their unique mesoporous structure, great specific volume and easy functionalisation. Moreover, MSN are biocompatible, non-toxic, and their cell uptake via endocytosis has been described.

Recently, mesoporous silica nanoparticles coated with lactose have been described for the controlled release of pharmaceuticals in the presence of β-galactosidase, found in the human body, especially in the enterocytes of the villi in the small intestine (A. Bernardos et al., Angew. Chem. Int. Ed., 2009, 48, 5884-5887). However, the release of the load in the system described would not be very selective in vivo, as the hydrolysis of just one β bond (1 → 4) would be sufficient to release the load in part. Therefore, a system of this type would have scant selectivity, as cells with a normal expression of the enzyme would cause the release. Also, the release of the load would be fast, preventing its distribution throughout the entire tissue to be treated.

Given the urgent need to develop new therapeutic ways of preventing the appearance of human diseases and failures related to senescence, there is a continuing need for systems for the selective treatment of senescent cells.

### DESCRIPTION OF THE INVENTION

The present invention relates to nanoparticles made of a mesoporous material for the controlled release of substances in senescent cells. The release of the load is activated by the presence of β-galactosidase associated to the senescence (SA-β-gal) in senescent cells. The existence of this SA-β-gal in these cells is explained by the overexpression of endogenous liposomal β-galactosidase which occurs specifically in senescent cells.

The present invention presents the following benefits:
- provides a more specific and controlled release
- the release occurs selectively in cells which overexpress β-galactosidase, such as senescent cells,
- the nanoparticles are introduced in the cells preferably through the lysosomal route, meaning that they travel directly to the cell region with the highest expression of β ―galactosidase.

A first aspect of the present invention relates to a nanodevice for the controlled release of substances which comprises a mesoporous support coated with oligosaccharides, wherein said oligosaccharides comprise at least 3 units of monosaccharides, and wherein all the monosaccharidesare galactose.

A second aspect of the present invention relates to the method for obtaining the nanodevice as described above which comprises the stages of:
a) functionalising the oligosaccharides with a silane group
b) suspending the support in a solution of the load to be released
c) adding to the above suspension an excess of the oligosaccharide functionalised in stage (a).

A third aspect of the present invention relates to a composition which comprises nanodevices such as those described above.

A fourth aspect of the present invention relates to the use of the nanodevice as described above, for the preparation of a medicament.

A fifth aspect of the present invention relates to the use of the nanodevice as described above, for the preparation of a medicament for the treatment and/or prevention of diseases which occur with overexpression of β-galactosidase.

A sixth aspect of the present invention relates to the use of the nanodevice as described above, for the manufacture of a cosmetic composition.

A seventh aspect of the present invention relates to the use of the nanodevice as described above, for the manufacture of equipment for the detection and quantification of senescent cells.

### DETAILED DESCRIPTION OF THE INVENTION

Throughout this description and the claims, the word "comprises" and its variants do not aim to exclude other technical characteristics, additives, components or steps. For persons skilled in the art, other objects, benefits and characteristics of the invention will be inferred partly from the description and partly from the practice of the invention.

A first aspect of the present invention relates to a nanodevice for the controlled release of substances which comprises a mesoporous support coated with oligosaccharides, wherein said oligosaccharides comprise at least 3 units of monosaccharides, and wherein all the monosaccharides are galactose. Preferably, the nanodevice comprises 3 to 10 units of monosaccharides and even more preferably 3 to 6 units of monosaccharides.

The term "nanodevice" is understood in the context of the invention to mean a device of nanometric size capable of releasing pharmaceuticals or cosmetics in a selective manner.

The term "oligosaccharide" is understood to mean a carbohydrate which consists of 3 to 15 units of monosaccharides (basic units of sugars, generally with the general formula CₙH₂ₙOₙ).

Also, in a preferred embodiment of the first aspect of the present invention, all the monosaccharides are galactose.

The nanodevices of the invention can have a diameter of 10 nm to 250 nm, preferably of 25 nm to 150 nm.

In another preferred embodiment of the first aspect of the present invention, the support is a mesoporous material, preferably mesoporous silica.

"Mesoporous support" is understood to mean a support which comprises pores with a diameter of between 0.01 nm and 50 nm. Similarly, mesoporous silica is understood to mean a silica with pores of the indicated diameter.

This mesoporous support preferably has a pore diameter of 0.5 nm to 10 nm, preferably of 1.5 nm to 3.5 nm.

In another preferred embodiment of the first aspect of the present invention, the nanodevice also comprises the substance to be released. This substance to be released can be selected from the list which comprises indicators, compounds for the reactivation of telomerase, cytotoxic compounds and any combinations thereof.

Indicator (or marker) is understood to mean a molecule which when released marks the cell in some way that allows it to be detected and/or quantified. These indicators do not have significant effects on cell viability. An example of an indicator would be fluorescent substances such as rhodamine-B, Ru(Bipy)₃²⁺, safranin and fluorescein.

Compound for the reactivation of telomerase means the molecule or biomolecule which when supplied to a cell produces an increase in telomerase activity, translating into an increase in the length of the telomeres and/or a maintenance in the size thereof. As a consequence, cell viability increases. Said effect may be achieved directly, by modulating the catalytic activity of the telomerase, or indirectly by increasing the levels of telomerase. Examples of compounds for the reactivation of telomerase valid in the field of the invention would be peptide GSE 24-2, and molecules TAT2 (cycloastragenol), TA-65 and GRN510.

Cytotoxic compounds means compounds which are toxic for cells. In contact with cytotoxic compounds, the cells may suffer necrosis, apoptosis or may cease to grow and divide. Examples of cytotoxic compounds valid in the context of the invention would be intercalating agents (among others cisplatin, oxaliplatin or carboplatin), nucleotide antagonists /inhibitors of the replication machinery (among others doxorubicin, camptothecin, etoposide, 5-fluorouracil, gemcitabine, cytosine, arabinoside or 6-mercaptopurine), inhibitors of the spindle apparatus (among others taxanes, vincristine, vinblastine or adriamycin) or inhibitors of folate synthesis (among others methotrexate or pemetrexed).

Preferably, the substance to be released is selected from the list which comprises peptide GSE 24-2, peptide TAT2 (cycloastragenol), cisplatin, oxaliplatin, carboplatin, doxorubicin, camptothecin, etoposide, 5-fluorouracil, gemcitabine, cytosine, arabinoside, 6-mercaptopurine, taxanes, vincristine, vinblastine, adriamycin methotrexate, pemetrexed and any combinations thereof.

These substances, selectively released can prevent and eliminate/replace or detect and mark senescent cells. Senescent cells present in a specific manner an overexpression of endogenous liposomal β-galactosidase. This β-galactosidase is capable of hydrolysing the oligosaccharides which coat the support, thus releasing the load.

In another preferred embodiment of the first aspect of the present invention, the proportion of the substance to be released is of 0.01 to 0.50 g per g of support.

A second aspect of the present invention relates to the method for obtaining the nanodevice as described above which comprises the stages of:
a) functionalising the oligosaccharides with a silane group
b) suspending the support in a solution of the load to be released
c) adding to the above suspension an excess of the oligosaccharide functionalised in stage (a).

Functionalising oligosaccharides with a silane group is understood to mean making one of the two ends, preferably the end which contains an anomeric carbon react with a silane functional group, which allows the oligosaccharides chain to anchor to the support, which is preferably made of silica.

Preferably, stage (b) is left to stir for 1 to 48 hours, preferably for 10 to 30 hours.

The mesoporous support may be obtained commercially or may be prepared prior to its use. Normally, to synthesise a material such as MCM-41 a mixture of n-cetylmethylammoniumbromide and NaOH is used, to which the tetraethyl orthosilicate (TEOS) is added drop by drop. Once washed and dried, the solid obtained is calcined in an oxidising atmosphere to eliminate the template phase.

In a preferred embodiment of the second aspect of the present invention stage (b) is carried out in polar solvents, preferably the solvent is selected from the group which comprises methanol, ethanol, propanol, isopropanol, acetone and acetonitrile. Excellent results have been obtained when the solvent is ethanol.

Once the support has been saturated with the substance to be released, it can proceed to be coated with the functionalised oligosaccharide. Preferably, the proportion of functionalised oligosaccharide / support is 1 to 5 times in mass (in other words, for every gram of support there are between 1 and 5 grams of functionalised polysaccharide) This stage (c) is left to stir preferably for 0.5 to 20 hours, more preferably for 1 to 10 hours.

A third aspect of the present invention relates to a composition which comprises nanodevices such as those described above. The composition, defined in a general manner, is a set of components which is made up of at least the product as it is described in the invention.

In a preferred embodiment of the third aspect of the present invention, the composition is pharmaceutical. This pharmaceutical composition preferably comprises pharmaceutically acceptable excipients. The pharmaceutical composition is a set of components which is made up of at least the product of the invention (the nanodevices along with the substance to be released) in any concentration, which has at least one application in improving the physical or physiological or psychological wellbeing of a subject, which implies an improvement in their general state of health.

For its application in therapy, the product of the invention will be provided, preferably, in a pharmaceutically acceptable or substantially pure form, in other words, which has a pharmaceutically acceptable level of purity, excluding normal pharmaceutical additives such as diluents and carriers, and not including material considered toxic at normal dosage levels. The levels of purity for the active principle are preferably higher than 50%, more preferably higher than 70%, and even more preferably higher than 90%. In a preferred embodiment, they are higher than 95% of the compound described above, or of its salts, solvates or pro-pharmaceuticals.

In the sense used in this description, the term "therapeutically effective quantity" refers to that quantity of the component of the pharmaceutical composition which when administered to a mammal, preferably a human, is sufficient to produce the prevention and/or treatment, as defined below, of a disease or pathological condition of interest in the mammal, preferably human. The therapeutically effective quantity will vary, for example, depending on the patient's age, body weight, general state of health, gender and diet; the mode and moment of administration; the speed of excretion, the combination of drugs; the severity of the specific disorder or pathological condition; and the subject undergoing therapy, but can be determined by a specialist in the technique based on their own knowledge.

The term medicament has a more limited meaning that the meaning of "pharmaceutical composition", as defined in the present invention, as the medicament necessarily implies a preventive or therapeutic effect, in other words, a physiological effect in the subject.

The term "excipient" refers to a substance which helps to absorb any of the components of the product of the invention, stabilises said components or helps to prepare the pharmaceutical composition in the sense of giving it consistency or adding flavours that make it more agreeable. Therefore, excipients could have the function of keeping the components together, such as for example starches, sugars or celluloses, the function of sweetening, function of colouring, function of protecting the medicament such as for example to isolate it from air and/or moisture, function of filling a tablet, capsule or any other form of presentation such as for example dibasic calcium phosphate, disintegrating function to facilitate the dissolution of the components and their absorption in the gut, without excluding other types of excipients not mentioned in this paragraph. Therefore, the term "excipient" is defined as that matter which, included in galenic forms, is added to the active principles or to their associations to enable its preparation and stability, modify its organoleptic properties or determine the physical and chemical properties of the pharmaceutical composition and its bioavailability. The "pharmaceutically acceptable" excipient must allow the activity of the compounds of the pharmaceutical composition, in other words, must be compatible with said components. Examples of excipients are binders, fillers, disintegrants, lubricants, coatings, sweeteners, flavourings and colourings. More specific non limiting examples of acceptable excipients are starches, sugars, xylitol, sorbitol, calcium phosphate, steroid fats, talcum, silica or glycerine among others.

The "galenic form or pharmaceutical form" is the arrangement the active principles and excipients adapt to in order to constitute a medicament. It is defined by the combination of the form in which the pharmaceutical composition is presented by the manufacturer and its form of administration.

The pharmaceutical composition can comprise a "vehicle" or carrier, which is preferably an inert substance. The function of the vehicle is to facilitate the incorporation of other compounds, allow for a better dosage and administration or give consistency and shape to the pharmaceutical composition. Therefore, the vehicle is a substance which is used to dilute any of the components of the pharmaceutical composition of the present invention up to a determined volume or weight; or which even without diluting said components is capable of allowing for a better dosage and administration or giving consistency and shape to the medicament. The vehicle is pharmaceutically acceptable. When the form of presentation is liquid, the pharmaceutically acceptable vehicle is the diluent.

Also, the excipient and the vehicle must be pharmacologically acceptable, in other words, the excipient and the vehicle must be permitted and evaluated in such a way that it does not cause harm to the organisms to which it is administered.

The pharmaceutical composition of the invention can comprise another active substance. In addition to the requirement of therapeutic efficacy, where said pharmaceutical composition may need to use other therapeutic agents, there may be other fundamental reasons which make it necessary or advisable to a great extent to use a combination of a compound of the invention and another therapeutic agent. The term "active principle" is any matter, irrespective of its origin, human, animal, vegetable, chemical or other type, which is attributed activity appropriate to constitute a medicament.

In each case the form of presentation of the medicament will adapt to the type of administration used, for this reason, the composition of the present invention may be presented in the form of solutions, or any other clinically allowed form of administration and in a therapeutically effective quantity. The pharmaceutical composition of the invention can be formulated in solid forms, semi-solid, liquid or gaseous, such as tablet, capsule, powder, granule, ointment, solution, suppository, injectable, inhalant, gel, syrup, nebuliser, microsphere or aerosol, preferably in the form of a tablet, capsule, powder, granule, solution, suppository or syrup.

The compositions mentioned above can be prepared using conventional methods, such as those described in the Pharmacopoeias of different countries and in other reference texts.

The compounds and compositions of the present invention can be used together with other medicaments in combined therapies. The other drugs can form part of the same composition or of another different composition, for administration at the same time or at different times.

In an embodiment of the third aspect of the present invention, the composition is cosmetic.

In the present invention "cosmetic" is understood to mean those preparations consisting of natural or synthetic substances or combinations thereof, for external use on the various parts of the human body: skin, hair system, nails, lips, outer genitalia, teeth and mucous membranes of the oral cavity, with the exclusive or main objective of cleaning them, perfuming, changing their appearance , protecting them or keeping them in good condition and/or correcting body odours but not to produce a therapeutic effect. The cosmetic composition can contain pharmaceutically and pharmacologically acceptable excipients and/or vehicles as described in previous paragraphs. On a separate note, the cosmetic composition can be presented in any form adapted to topical administration, preferably in the form of a cream, lotion, gel or powder.

A fourth aspect of the present invention relates to the use of the nanodevice as described above, for the preparation of a medicament.

The medicament referred to in the present invention may be for human or veterinary use. The "medicament for human use" is any substance or combination of substances which is presented as having properties for the treatment or prevention of diseases in human beings or which can be used in human beings or administered to human beings for the purpose of restoring, correcting or modifying physiological functions, by exerting a pharmacological, immunological, or metabolic action or establishing a medical diagnosis. The "medicament for veterinary use" is any substance or combination of substances which is presented as having healing or preventive properties in relation to animal diseases or which can be administered to the animal for the purpose of restoring, correcting or modifying its physiological functions by exerting a pharmacological, immunological, or metabolic response, or establishing a veterinary diagnosis. "Pre-mixtures for animal feed" prepared for adding to a feed will also be considered "veterinary medicaments".

A fifth aspect of the present invention relates to the use of the nanodevice as described above, for the preparation of a medicament for the treatment and/or prevention of diseases which occur with overexpression of β-galactosidase, preferably this overexpression takes place in senescent cells. Preferably, the disease is selected from the list that comprises the Wiedemann-Rautenstrauch syndrome of neonatal progeria, the Werner syndrome of adult progeria, Hutchinson-Gilford syndrome, Rothmund Thompson syndrome, Mulvill-Smith syndrome, Cockayne syndrome, Dyskeratosis Congenita, idiopathic pulmonary fibrosis, aplastic anaemia, emphysema, type 2 diabetes, and degeneration of cartilage

A sixth aspect of the present invention relates to the use of the nanodevice as described above, for the manufacture of a cosmetic composition.

A seventh aspect of the present invention relates to the use of the nanodevice as described above, for the manufacture of equipment for the detection and quantification of senescent cells. When the substance to be released is an indicator, the senescent cells will be marked for their detection and/or quantification. In this way, the degree of ageing and/or tissue degeneration can be evaluated.

Next the invention is illustrated by means of tests conducted by the inventors, which reveal the selective release of substances in senescent cells. The following examples and figures are provided by way of illustration and are not intended to limit the present invention.

### BRIEF DESCRIPTION OF THE DRAWINGS

**FIG. 1****. Synthesis of the derived galactooligosaccharide (I).**
**FIG. 2****. X-ray diffractogram of the mesoporous silica and Transmission Electron Microscopy (TEM) images of the nanodevices of the invention loaded with Rhodamine-B (S1) and calcined MCM-41 support;** I: Intensity, 2θd: 2θ degrees, S1: nanodevices loaded with Rhodamine coated with galactooligosaccharides (I), MCM-41 c: calcined mesoporous silica nanoparticles, MCM-41 m: non-calcined mesoporous silica nanoparticles, nm: nanometres.

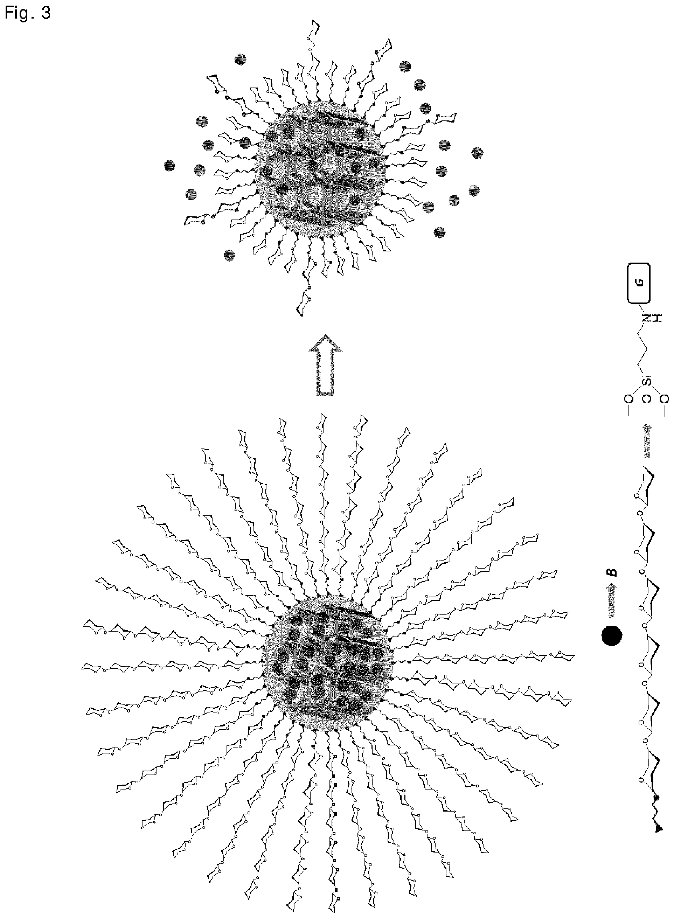

**FIG. 3****. Graph of release of the Rhodamine-B in the presence of β-gal;** B: Rhodamine B; G: galactooligosaccharide.

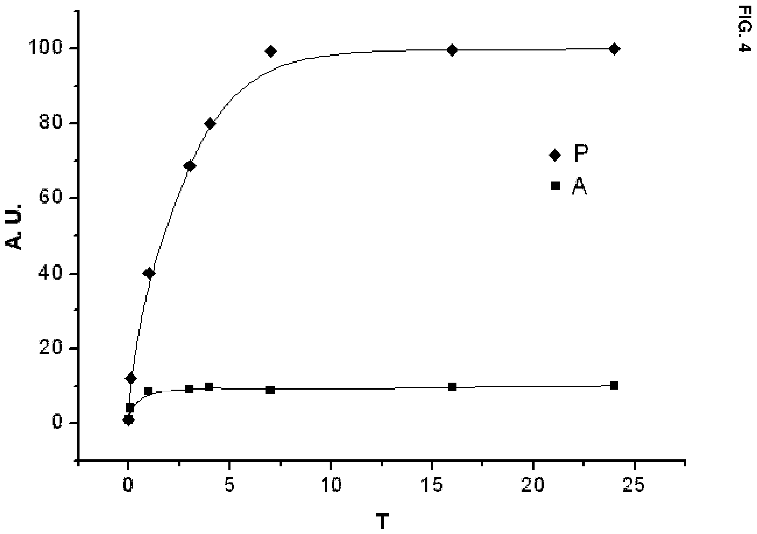

**FIG. 4****. Release profiles of Rhodamine-B of S1 in the absence and presence of β-gal enzyme in water at pH 7.5 and room temperature;** P: presence of β-gal; A: absence of β-gal; A.U.: Fluorescence intensity in %; T: time (hours).
**FIG. 5****.** % **Cell viability in the presence of the nanoparticles of the invention.** A: % Viability of the yeast cells in the presence of the indicated quantities of **S1**; B: % Viability of the H460 cells after 48 hours of treatment with the indicated quantities of **S1**; %V: % Viability.

### EXAMPLES

### Example 1 Synthesis of the silane derivative of the oligosaccharide.

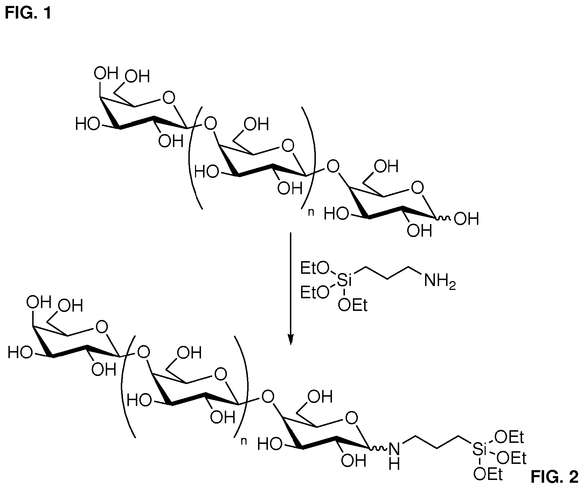

The galactooligosaccharide (I) (fig. 1, n= 3 -6) was obtained as a syrup with a pH of 3.8. This syrup was diluted in water and the pH was increased up to 7 with NaHCO₃. Then, the solution was lyophilised obtaining a white solid. Afterwards, 5 grams of the solid were dissolved in 100 ml of anhydrous ethanol, and a solution of 3-aminopropyltriethoxysilane (2, 5.85 ml, 25 mmol) was added. The reaction mixture was stirred for 24 h at room temperature. The solvent was evaporated under reduced pressure to obtain a white solid (I). Fig. 1.
¹H NMR (300 MHz, D₂O): 0.42 (t, 2H, -C**H₂**-Si-), 1.02 (t, 9 H, C**H₃**-CH₂-O-Si-), 1.53 (m, 2H, -C**H₂**-CH₂-Si-), 2.74 (t, 2H, NH-C**H₂**-CH₂-CH₂-Si-), 3.20-3.77 (m, nH, galactooligosaccharide, CH₃-C**H**₂-O-Si-), 5.13 (d, 1 H, -O-C**H**-O-) ppm, ¹³C NMR (300 MHz, D₂O): 9.62 (-**C**H₂-Si-), 16.65 (**C**H₃-CH₂-O-Si-), 21.74 (-**C**H₂-CH₂-Si-), 41.96 (-NH-**C**H₂-CH₂-CH₂-Si-), 57.25 (CH₃-**C**H₂-O-Si-), 61.84 (HO-**C**H₂-CH-), 72.67-78.11 (HO-CH-), 89.57 (-O-**C**H-CH), 94.84 (-O-**C**H-NH-), 100.25 (-O-CH-O-) ppm.

### Example 2 Synthesis of the mesoporous support.

The mesoporous support that was used was mesoporous silica, specifically MCM-41.

N-cetyltrimethylammoniumbromide (CTABr, 2.00 g, 5.48 mmol) was dissolved in 960 ml of deionised water. NaOH (ac) (2.00 M, 7.00 ml) was added to the CTABr solution. Next the temperature was adjusted to 95ºC. Tetraethyl orthosilicate (TEOS,10.00 ml, 5.14*10⁻² mol) was then added drop by drop to the previous solution. The mixture was left to stir for 2.5 h and a white precipitate was obtained. The solid product was centrifuged and washed with deionised water until neutral pH and dried at 60ºC for 13h. To prepare the final porous product MCM-41, the dry solid product was calcined at 550ºC in an oxidising atmosphere for 5 h to remove the surfactant, also known as the structure directing agent.

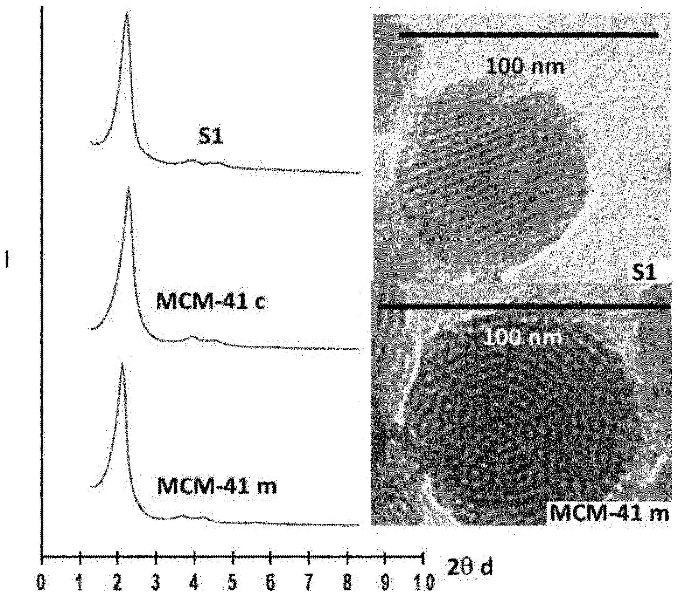

The X-ray diffractogram (XRD) of the MCM-41 material (fig. 2) shows four small angle reflections typical of the hexagonal geometry that can be indexed as Bragg peaks (100), (110), (200) and (210). A significant displacement in the reflection (100) can be clearly appreciated and a widening of the peaks (110) and (200) in the XRD in powder of the calcined MCM-41 sample. This corresponds to a unit cell contraction of about 6-8 Å due to the condensation of silanols during the calcination stage. In spite of this clear partial loss of order, observation of the reflections (100) and (200) indicates that certain relative mesoporous symmetry is preserved after calcination. The Na₂ adsorption desorption isotherms of the mesoporous nanoparticles shows a typical type IV curve with a specific surface of 999.6 m²g⁻¹, and a pore volume of 1.17 cm³g⁻¹. From the XRD, porosimetry, and Transmission Electron Microscopy (TEM) studies the ao parameter of the hexagonal cell (4.43 nm), pore diameter (2.45 nm) and wall thickness value (1.98 nm) were calculated.

### Example 3. Synthesis of the nanodevices of the invention loaded with Rhodamine-B (fluorescent substance).

50 mg of MCM-41 and 19 mg of Rhodamine-B (0.04 mmol) were suspended in 10 ml of ethanol in a round bottom flask in an inert atmosphere. The mixture was left stirring for 24 h at room temperature with the objective of achieving maximum load in the pores of MCM-41. Then, an excess of (I), silane derivative of the galactooligosaccharide, (100 mg in 10 ml of ethanol) were added and the final mixture was left stirring for 5.5 h at room temperature. Finally, the nanodevices of the invention loaded with Rhodamine-B (S1) were filtered and washed with water.

The reflections (110) and (200) were partially lost, certainly because of a reduction in the contrast due to the fact that the empty space of the pores was filled with the Rhodamine-B. Also, the preservation of the mesoporous structure in the final functionalised solids was confirmed by TEM.

To determine the content of Rhodamine-B in the final hybrid material, 30 mg of **S1** was suspended in 75 ml of water at pH 7.5 in the presence of 7500 ppm of β-galactosidase. The mixture was left stirring at room temperature for 72 h to achieve the full release of the Rhodamine-B. Afterwards, the suspension was centrifuged and the supernatant was isolated and dried at reduced pressure. The crude obtained was dissolved in acetone and filtered. The solution obtained was dried and the residue was dissolved in 4 ml of ethanol. The final content of Rhodamine-B was determined by means of a calibration curve based on the increase in the absorption band centred in 550 nm with the concentration, obtaining 0.14 g/gSiO₂ in weight.

The content of the galactooligosaccharide (**I**) in **S1** was calculated through thermogravimetric analysis, 0.28 g/gSiO₂ in weight.

### Example 4. Load release studies.

10 mg of **S1** were suspended in 18.75 ml of water at pH 7.5 and next 6.25 ml of a lactozyme enzyme solution (β-D-galactosidase) were added (0.3 g of enzyme in 10 ml of water at pH 7.5). The release of the Rhodamine-B of the pores to the aqueous solution was monitored through the emission spectrum of Rhodamine-B centred in 580 nm.

### Example 5. Experiments of load release in S. cerevisiae.

### Strains and culture conditions

All the strains of yeast used in this study have the genetic base of BY4741 (MATα leu2Δ0, his3Δ0, met15Δ0, ura3Δ0). The β-Gal^{oe} strain was generated by transforming the WT BY4741 strain with expression vector β-gal p477 (URA3). Standard methods were used for the culture and manipulation of the yeasts. The minimum medium (SD) contained 2% glucose, 0.67% of yeast nitrogen base and the amino acid supplements appropriate for maintaining the selection of URA3. The yeast cultures were maintained at 30ºC.

### Fluorescence microscope and cell viability studies

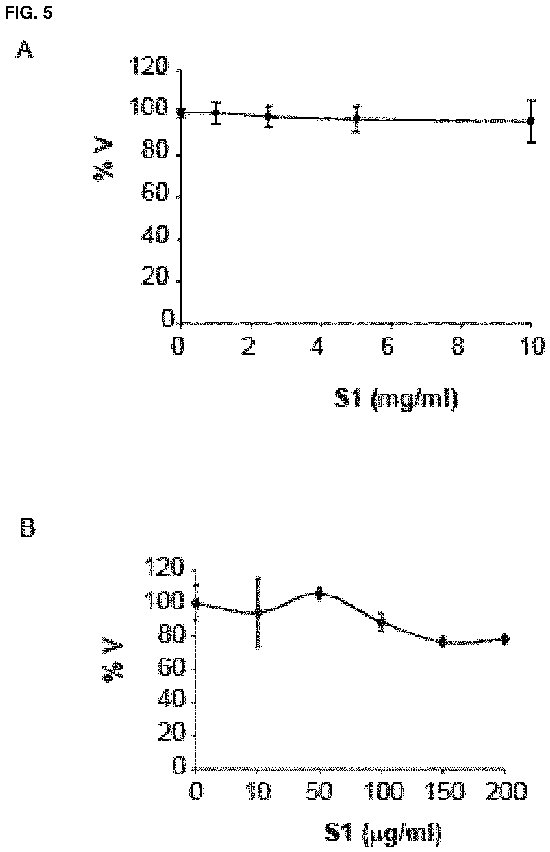

The BY4741 (WT, *wild type*) and β-Gal^{oe} yeasts were cultured in SD medium overnight at 28ºC stirring continuously in a density of 10⁸ cells/ml. Then, aliquotes of 1 ml of this suspension were centrifuged and re-suspended in 100 µl of SD medium at pH 3.7 containing 5 mg/ml of **S1** nanoparticles loaded with Rhodamine-B and were incubated for 6 h at 40ºC. The intracellular release was monitored by fluorescent microscopy using a NIKON ECLIPSE 600 microscope equipped with a fluorescent lamp NIKON Y-FL, λₑₓ= 540 nm, λₑₘ= 650 nm. After the incubation period, approximately 300 cells were seeded in a YPD plate and were incubated for 72 h. Finally, the formation of colonies was quantified and cell viability was calculated. The experiments were repeated twice containing triplicates. The data is expressed as (mean ± S.D., standard deviation). The incubation with **S1** nanoparticles did not affect cell viability throughout the experiment (table 1, fig. 5 A)

**Table 1. % Viability of β-Gal^{oe} yeast incubated with S1.**

| S1 (mg/ml) | % Viability | S.D. |
|---|---|---|
| 0 | 100 | 2 |
| 1 | 100 | 5 |
| 2.5 | 98 | 5 |
| 5 | 97 | 6 |
| 10 | 96 | 10 |

Fluorescence was only detected in the β-Gal^{oe} yeast cells with great β-gal activity. In these conditions the percentage of internalisation in β-Gal^{oe}, i.e., % of cells stained with the Rhodamine B colouring is 85% with an S.E. of 5% (p<0.01) for 5 mg/ml of S1.

| **yeast** | **β-Galactosidase Activity (enzymatic units)** | **S.D.** |
|---|---|---|
| WT | 2.0000 | 1.0000 |
| -Galoe | 32.0000 | 2.0000 |

### Example 6. Experiments of load release in human cells

### Cell culture

The reagents for the tissue culture were obtained from GIBCO/lnvitrogen Corporation/Life Technologies Life Sciences. Dermal fibroblasts from two patients with X-linked Dyskeratosis congenita (X-DC) (X-DC1774 and X-DC4646) and control fibroblasts with a high number of passages (DC1787) were obtained from the Coriell Cell Repository. The X-DC1774 and X-DC4646 fibroblasts belong to males with mutations c.109_11delCTT, c.385A>T and c.5C>T *DKC1* respectively. The DC17871 control fibroblasts belong to a woman carrier of mutation c.109_11delCTT *DKC1.* The H460 cells (non-small cell lung cancer cells expressing telomerase) were obtained from ATCC and were maintained in RMPM supplemented with 10% foetal bovine serum (FBS) and the primary human cells were maintained in MEM (*Minimum Essential Medium*, GIBCO) supplemented with 15% FBS at 37ºC and 5% of CO₂. The culture medium was supplemented with gentamicin (56 µg/ml and L-glutamine (2mM).

### Analysis of senescence by fluorescent microscopy in vivo.

Control and X-DC (1*10⁴ cells) fibroblasts were placed on 6 plates of wells and were fixed after 4 days to test the activity of the β-galactosidase SA-β-gal (Senescence Detection Kit, BioVision, USA). The location of the load release of the nanoparticles was visualised using fluorescent microscopy. To this end, the cells were cultured in ibiTreat (Ibidi) microscopy chambers of eight wells with plates of 1µ and were treated with the mesoporous particles **S1.** The fluorescence was visualised by means of *in vivo* microscopy during 48 h in a Microscope Observer Z1. Image acquisition and processing was carried out as follows: type, numerical aperture and magnification of the objective lenses: APOCHROMAT Plan by Zeiss 20x, Temperature 37ºC and 5% CO₂. Camera: Cascade 1k. Acquisition software: Axiovision 4.8.

The percentage of senescent cells (blue) was calculated in the images by bright field microcopy at 100x magnification (Nikon Eclipse TS100 Microscopy, USA). The following values were obtained:

**Table 2. % of senescent cells.**

| **Cells** | **% of senescent cells** |
|---|---|
| DC1787 | 35.5 |
| X-DC1774 | 55.0 |
| X-DC4646 | 65.00 |
| H460 | 0.3 |

### Cell viability assays

The viability of H460 and DC1787 in the presence of **S1** was determined using a crystal violet staining method. The cells were cultured in 24-well plates, treated with different quantities of mesoporous silica particles and 48 h after treatment were fixed with 1% glutaraldehyde, were washed with PBS and the remaining cells were stained with 0.1% crystal violet. The colorimetric assay using 595 nm Elisa was used to estimate the number of cells per well. The experiments were repeated 3 times in quadruplicate.

The assays showed that the nanoparticles of the invention are not toxic in human cells and in yeasts (fig. 5 B).

### Example 7. Control experiment

To verify that the lack of Rhodamine-B stain in the H460 cells was not an artefact, nanoparticles loaded with Rhodamine-B were prepared and coated withhydrolysed starch (**S2**). These nanoparticles are similar to **S1** but contain an oligosaccharide capable of hydrolysing by the amylase in lysosomes. The **S2** nanoparticles were synthesised following a similar method to the one described, using mesoporous silica as support, Rhodamine B as load and hydrolysed starch (Glucidex® 47) as coating.

When **S2** was used in the internalisation studies under similar conditions to those described above, a clear staining was detected in all cell lines used, indicating that the release of S1 was clearly dependent on the presence of β-galactosidase.

## Claims

1. Nanodevice for the controlled release of substances which comprises a support coated with oligosaccharides, wherein said oligosaccharides comprise at least 3 units of monosaccharides, and wherein all of the monosaccharides are galactose.

2. The nanodevice according to the preceding claim which comprises 3 to 10 units of monosaccharides, preferably the oligosaccharide comprises 3 to 6 units of monosaccharides.

3. The nanodevice according to any of the preceding claims **characterised in that** it has a diameter of 10 nm to 250 nm, preferably of 25 nm to 150 nm.

4. The nanodevice according to any of the preceding claims wherein the support is a mesoporous support, preferably mesoporous silica.

5. The nanodevice according to any of the preceding claims wherein the pore diameter of the support is 0.5 nm to 10 nm, preferably 1.5 nm to 3.5 nm.

6. The nanodevice according to any of the preceding claims which moreover comprises the substance to be released is selected from the list which comprises indicators, compounds for the reactivation of telomerase, cytotoxic compounds and any combinations thereof, preferably the substance to be released is selected from the list which comprises peptide GSE 24-2, peptide TAT2, cisplatin, oxaliplatin, carboplatin, doxorubicin, camptothecin, etoposide, 5-fluorouracil, gemcitabine, cytosine, arabinoside, 6-mercaptopurine, taxanes, vincristine, vinblastine, adriamycin methotrexate, pemetrexed and any combinations thereof.

7. The nanodevice according to claim 6 wherein the proportion of the substance to be released is of 0.01 to 0.50 g per g of support.

8. Process of obtainment of the nanodevice according to any of the preceding claims which comprises the stages of:
a) functionalising the oligosaccharides with a silane group,
b) suspending the support in a solution of the substance to be released,
c) adding to the above suspension an excess of the oligosaccharide functionalised in stage (a).

9. Composition which comprises the nanodevices according to claims 1 to 7.

10. The composition according to the preceding claim, wherein said composition is pharmaceutical, preferably moreover comprises pharmaceutically acceptable excipients, more preferably the composition is in the form of a tablet, capsule, powder, granule, solution, suppository or syrup.

11. The composition according to any of claim 9, wherein said composition is cosmetic, preferably the composition is in the form of a cream, lotion, gel or powder.

12. A nanodevice according to claims 1 to 7, for use as medicament.

13. A nanodevice according to claims 1 to 7, for use in the treatment and/or prevention of diseases which occur with overexpression of β-galactosidase selected from the list that comprises the Wiedemann-Rautenstrauch syndrome of neonatal progeria, the Werner syndrome of adult progeria, Hutchinson-Gilford syndrome, Rothmund Thompson syndrome, Mulvill-Smith syndrome, Cockayne syndrome, Dyskeratosis Congenita, idiopathic pulmonary fibrosis, aplastic anaemia, emphysema, type 2 diabetes, and degeneration of cartilage, preferably the overexpression of β-galactosidase takes place in senescent cells.

14. Use of the nanodevice according to claims 1 to 7 for the detection and quantification of senescent cells.

## Patentansprüche

1. Nanovorrichtung zur kontrollierten Freisetzung von Stoffen, die einen mit Oligosacchariden beschichteten Träger umfasst, wobei die Oligosaccharide mindestens 3 Einheiten von Monosacchariden umfassen und wobei alle Monosaccharide Galactose sind.

2. Nanovorrichtung nach dem vorhergehenden Anspruch, die 3 bis 10 Einheiten von Monosacchariden umfasst, wobei das Oligosaccharid vorzugsweise 3 bis 6 Einheiten von Monosacchariden umfasst.

3. Nanovorrichtung nach einem der vorhergehenden Ansprüche, **dadurch gekennzeichnet, dass** sie einen Durchmesser von 10 nm bis 250 nm, vorzugsweise von 25 nm bis 150 nm aufweist.

4. Nanovorrichtung nach einem der vorhergehenden Ansprüche, wobei der Träger ein mesoporöser Träger, vorzugsweise mesoporöses Siliciumdioxid ist.

5. Nanovorrichtung nach einem der vorhergehenden Ansprüche, wobei der Porendurchmesser des Trägers 0,5 nm bis 10 nm, vorzugsweise 1,5 nm bis 3,5 nm beträgt.

6. Nanovorrichtung nach einem der vorhergehenden Ansprüche, die darüber hinaus den freizusetzenden Stoff umfasst, der ausgewählt ist aus der Liste, die Indikatoren, Verbindungen zur Reaktivierung von Telomerase, zytotoxische Verbindungen und beliebige Kombinationen davon, wobei der freizusetzende Stoff vorzugsweise ausgewählt ist aus der Liste, die Peptid GSE 24-2, Peptid TAT2, Cisplatin, Oxaliplatin, Carboplatin, Doxorubicin, Camptothecin, Etoposid, 5-Fluorouracil, Gemcitabin, Cytosin, Arabinosid, 6-Mercaptopurin, Taxane, Vincristin, Vinblastin, Adriamycin, Methotrexat, Pemetrexed und beliebige Kombinationen davon umfasst.

7. Nanovorrichtung nach Anspruch 6, wobei der Anteil des freizusetzenden Stoffs 0,01 bis 0,50 g pro g Träger beträgt.

8. Verfahren zum Erhalten der Nanovorrichtung nach einem der vorhergehenden Ansprüche, das die folgenden Stufen umfasst:
a) Funktionalisieren der Oligosaccharide mit einer Silangruppe,
b) Suspendieren des Trägers in einer Lösung des freizusetzenden Stoffs,
c) Zugeben eines Überschusses des in Stufe (a) funktionalisierten Oligosaccharids zu der obigen Suspension.

9. Zusammensetzung, welche die Nanovorrichtungen nach einem der Ansprüche 1 bis 7 umfasst.

10. Zusammensetzung nach dem vorhergehenden Anspruch, wobei die Zusammensetzung pharmazeutisch ist, vorzugsweise darüber hinaus pharmazeutisch verträgliche Hilfsstoffe umfasst, die Zusammensetzung mehr bevorzugt in Form einer Tablette, Kapsel, eines Pulvers, eines Granulats, einer Lösung, eines Zäpfchens oder eines Sirups vorliegt.

11. Zusammensetzung nach einem der Ansprüche 9, wobei die Zusammensetzung kosmetisch ist, wobei die Zusammensetzung vorzugsweise in Form einer Creme, Lotion, eines Gels oder eines Puders vorliegt.

12. Nanovorrichtung nach den Ansprüchen 1 bis 7 zur Verwendung als Medikament.

13. Nanovorrichtung nach den Ansprüchen 1 bis 7 zur Verwendung bei der Behandlung und/oder Vorbeugung von Krankheiten, die bei einer Überexpression von β-Galactosidase auftreten, ausgewählt aus der Liste, die das Wiedemann-Rautenstrauch-Syndrom der neonatalen Progerie, das Werner-Syndrom der adulten Progerie, das Hutchinson-Gilford-Syndrom, das Rothmund-Thompson-Syndrom, das Mulvill-Smith-Syndrom, das Cockayne-Syndrom, Dyskeratosis Congenita, idiopathische Lungenfibrose, aplastische Anämie, Emphysem, Typ-2-Diabetes und Knorpeldegeneration umfasst, wobei die Überexpression von β-Galactosidase vorzugsweise in seneszenten Zellen stattfindet.

14. Verwendung der Nanovorrichtung nach den Ansprüchen 1 bis 7 zum Nachweis und zur Quantifizierung von seneszenten Zellen.

## Revendications

1. Nanodispositif pour la libération contrôlée de substances qui comprend un support enrobé d'oligosaccharides, dans lequel lesdits oligosaccharides comprennent au moins 3 unités de monosaccharides, et dans lequel tous les monosaccharides sont du galactose.

2. Nanodispositif selon la revendication précédente qui comprend 3 à 10 unités de monosaccharides, de préférence l'oligosaccharide comprend 3 à 6 unités de monosaccharides.

3. Nanodispositif selon l'une quelconque des revendications précédentes **caractérisé en ce qu'**il a un diamètre de 10 nm à 250 nm, de préférence de 25 nm à 150 nm.

4. Nanodispositif selon l'une quelconque des revendications précédentes dans lequel le support est un support mésoporeux, de préférence de la silice mésoporeuse.

5. Nanodispositif selon l'une quelconque des revendications précédentes, dans lequel le diamètre des pores du support est de 0,5 nm à 10 nm, de préférence de 1,5 nm à 3,5 nm.

6. Nanodispositif selon l'une quelconque des revendications précédentes qui comprend en outre la substance à libérer qui est choisie dans la liste qui comprend des indicateurs, des composés pour la réactivation de la télomérase, des composés cytotoxiques et toute combinaison de ceux-ci, de préférence, la substance à libérer est choisie dans la liste qui comprend du peptide GSE 24-2, du peptide TAT2, du cisplatine, du oxaliplatine, du carboplatine, de la doxorubicine, de la camptothécine, du étoposide, du 5-fluorouracile, de la gemcitabine, de la cytosine, du arabinoside, de la 6-mercaptopurine, des taxanes, de la vincristine, de la vinblastine, de la adriamycine méthotrexate, du Pemetrexed et toute combinaison de ceux-ci.

7. Nanodispositif selon la revendication 6 dans lequel la proportion de la substance à libérer est de 0,01 à 0,50 g par g de support.

8. Procédé d'obtention du nanodispositif selon l'une quelconque des revendications précédentes qui comprend les étapes de :
a) fonctionnaliser les oligosaccharides avec un groupement silane,
b) mettre en suspension le support dans une solution de la substance à libérer,
c) ajouter à la suspension précédente un excès de l'oligosaccharide fonctionnalisé à l'étape (a).

9. Composition qui comprend les nanodispositifs selon les revendications 1 à 7.

10. Composition selon la revendication précédente, dans laquelle ladite composition est pharmaceutique, qui de préférence comprend en outre des excipients pharmaceutiquement acceptables, plus préférentiellement la composition se présente sous la forme d'un comprimé, une gélule, de la poudre, un granule, une solution, un suppositoire ou du sirop.

11. Composition selon l'une quelconque revendication 9, dans laquelle ladite composition est cosmétique, de préférence la composition se présente sous la forme d'une crème, une lotion, du gel ou de la poudre.

12. Nanodispositif selon les revendications 1 à 7, pour son utilisation en tant que médicament.

13. Nanodispositif selon les revendications 1 à 7, pour une utilisation dans le traitement et/ou la prévention de maladies qui surviennent avec une surexpression de la β-galactosidase choisie dans la liste qui comprend le syndrome progéroïde néonatal de Wiedemann-Rautenstrauch, le syndrome de Werner de progéria de l'adulte, le syndrome de Hutchinson-Gilford, le syndrome de Rothmund Thompson, le syndrome de Mulvill-Smith, le syndrome de Cockayne, la dyskératose congénitale, la fibrose pulmonaire idiopathique, l'anémie aplasique, l'emphysème, la diabète de type 2 et la dégénérescence du cartilage, de préférence, la surexpression de la β-galactosidase a lieu dans les cellules sénescentes.

14. Utilisation du nanodispositif selon les revendications 1 à 7 pour la détection et la quantification de cellules sénescentes.
